# FASCICULE DE BREVET EUROPEEN

(11) **EP 1 036 053 B1**
(45) Date de publication et mention de la délivrance du brevet: **05.03.2003**
(21) Numéro de dépôt: 98955717.8
(22) Date de dépôt: 23.11.1998
(51) Int. Cl.: C07C 13/42, C07C 7/04

(54) **PROCEDE DE PURIFICATION DU NORBORNENE PAR DISTILLATION**
VERFAHREN ZUR REINIGUNG VON NORBORNEN DURCH DESTILLATION
METHOD FOR PURIFYING NORBORNENE BY DISTILLATION

(30) Priorité: 02.12.1997 FR 9715161
(43) Date de publication de la demande: 20.09.2000
(73) Titulaire: Atofina, 92800 Puteaux (FR)
(72) Inventeur: KOTWICA, Roland, F-60700 Pontpoint (FR); MARBACH, André, F-60550 Verneuil en Halatte (FR)
(74) Mandataire: Neel, Henry
(86) Numéro de dépôt international: FR9802495
(87) Numéro de publication internationale: WO99028279

(56) Documents cités:
- DD-A- 215 526
- DD-A- 237 987
- US-A- 3 007 977

## Description

La présente invention porte sur un procédé de purification du norbornène par distillation.

La synthèse du norbornène à partir de dicyclopentadiène (DCPD) ou de cyclopentadiène (CPD) et d'éthylène donne un norbornène brut contenant des impuretés telles que le DCPD, le diméthanooctahydronaphtalène (DMON) ou le CPD (ces impuretés seront décrites plus en détail ci-après). La séparation de ces impuretés à partir du norbornène est compliquée du fait des nombreuses réactions équilibrées existant entre ces produits : toute distillation d'un brut de synthèse conduit à une distillation réactive.

Le brevet allemand DD 237 987 décrit un procédé de purification comprenant une distillation simple d'un brut issu de la réaction entre l'éthylène et CPD ou DCPD.

Le brevet américain US-A-3 007 977 décrit un procédé de purification du norbornène à partir d'un mélange réactionnel obtenu par réaction de l'éthylène avec le CPD et le DCPD, ledit mélange réactionnel comprenant du CPD n'ayant pas réagi et du norbornène. Ce procédé comprend les étapes consistant à :
- dans une première zone de fractionnement, fractionner ledit mélange réactionnel pour obtenir, en tant que fraction intermédiaire, du norbornène avec des quantités mineures de CPD, et rejeter le DCPD de ladite zone de fractionnement ;
- dans une seconde zone de fractionnement, fractionner ladite fraction intermédiaire en une fraction de tête comprenant du CPD et du norbornène et en une fraction comprenant essentiellement du norbornène,

Conformément à ce brevet américain US-A-3 007 977, il est donc fait appel, pour la purification d'un brut de synthèse du norbornène, à un système basé sur deux colonnes de distillation ; ce système est un système de base dans lequel le norbornène est successivement équeuté, puis étêté.

L'équilibre DCPD/CPD rend cependant un tel système peu efficace. En effet, le CPD formé dans la première colonne lors du chauffage de la charge se retrouve dans la fraction de tête. La température de la seconde colonne de distillation étant plus faible, la réaction en retour conduit à un norbornène contenant des lourds résiduels, tels que DCPD ou DMON. De plus, le produit obtenu dans ce pied de colonne est coloré, ce qui en limite les applications.

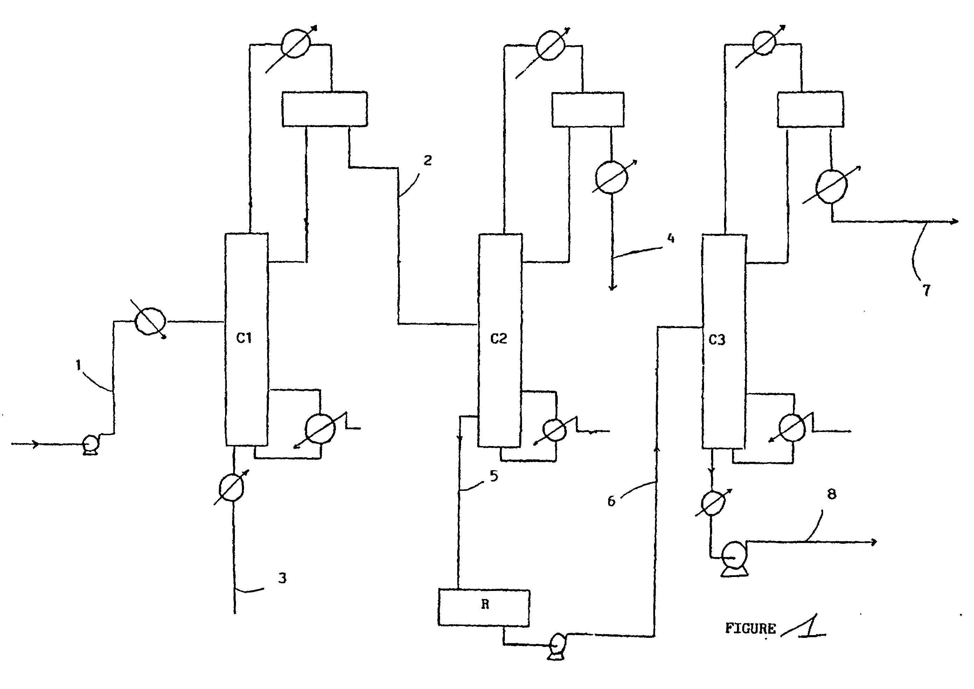

La demande de brevet français FR-A-2 438 639 décrit un procédé de fabrication du norbornène à partir des produits de la réaction de synthèse de norbornène entre DCPD ou CPD et éthylène :
- conformément à un premier mode de réalisation (illustré par la Figure 1 de FR-A-2 438 639), on introduit le brut de synthèse dans une première colonne de distillation, dont le produit de tête est, après condensation, mis à réagir à 70°C avec un temps de séjour moyen d'une heure pour dimériser le CPD en DCPD, puis réintroduit dans la colonne. Le mélange des produits qui tombe dans le pied de colonne à 105°C est introduit dans une seconde colonne de distillation, le produit contenant le norbornène étant vaporisé et tous les composés lourds étant soutirés comme produit de pied de colonne ;
- conformément à un second mode de réalisation (illustré par la Figure 2 de FR-A-2 438 639), on introduit le brut de synthèse dans une première colonne de distillation, où, à 112°C et avec un temps de séjour moyen d'environ 30 heures, une grande partie du produit contenant le norbornène est vaporisée, les composants lourds étant soutirés comme produits de pied de colonne et contenant du norbornène dans une très grande proportion. Le produit de tête est amené dans une seconde colonne de distillation, où l'on obtient en tête, du norbornène contenant encore des fractions de dicyclopentadiène, et, en pied, du norbornène de haute pureté.

L'inconvénient des deux systèmes décrits ci-dessus, sans étêtage, réside dans la présence d'impuretés légères, telles que l'isoprène, dont l'origine est le DCPD de départ, impuretés qui ne sont pas séparées du norbornène. Cette présence d'impuretés oblige à utiliser des qualités spéciales de DCPD si l'on désire obtenir du norbornène de haute pureté.

Dans les procédés selon US-A-3 007 977 et FR-A-2 438 639, la pureté obtenue pour le norbornène est fonction, non seulement des taux de reflux et des températures, mais encore de la charge globale des colonnes. En effet, la monomérisation du DCPD n'est pas immédiate, si bien que la composition est influencée également par les temps de séjour des produits dans les colonnes.

La présente invention a pour but de proposer un procédé de purification du norbornène par distillation qui ne comporte plus aucun des inconvénients des procédés de la technique antérieure et qui permette d'opérer industriellement pour l'obtention d'un norbornène de qualité satisfaisante.

Conformément à la présente invention, ce but est atteint, autrement dit, une qualité régulière de norbornène la plus exempte possible de lourds et de légers résiduels peut être obtenue, sans perte excessive de produit, par un procédé mettant en jeu trois colonnes de distillation successives.

Le procédé selon la présente invention, pour la purification du norbornène obtenu par la réaction du dicyclopentadiène ou du cyclopentadiène et de l'éthylène, le mélange brut réactionnel contenant :
- des impuretés légères, dont les températures d'ébullition sont inférieures à celle du norbornène ;
- des impuretés mi-lourdes, dont les températures sont comprises entre celle du norbornène et celle de l'éthyl norbornène ; et
- des impuretés lourdes, dont les températures sont supérieures à celles de l'éthyl norbornène, celui-ci étant par ailleurs inclus dans lesdites impuretés lourdes,
est caractérisé par le fait qu'on effectue une première distillation du mélange brut réactionnel dans une colonne d'équeutage (C1), éliminant une partie des impuretés lourdes et une partie des impuretés mi-lourdes ; puis une seconde distillation du mélange brut ainsi équeuté dans une colonne d'étêtage (C2), éliminant les impuretés légères ; et ensuite une troisième distillation du mélange ainsi étêté dans une colonne d'équeutage (C3), éliminant le reste des impuretés lourdes et mi-lourdes.

On peut par ailleurs poursuivre, dans un réservoir (R) interposé entre le colonne d'étêtage (C2) et la colonne de second équeutage (C3), la recombinaison du cyclopentadiène en dicyclopentadiène qui commence dans la colonne d'équeutage (C1) et se poursuit dans la colonne d'étêtage (C2).

De façon particulièrement préférée, on recycle à l'entrée de la colonne de premier équeutage (C1) le flux de pied de la colonne de second équeutage (C3).

On va maintenant décrire plus en détail les caractéristiques et le fonctionnement d'ensemble des trois colonnes de distillation (C1), (C2) et (C3), avec référence à la Figure unique du dessin annexé qui est un schéma général de l'installation. Les pourcentages sont donnés en poids.

### Colonne (C1)

Cette colonne, garnie d'éléments de remplissage, a pour but d'éliminer une partie des lourds et des mi-lourds; elle est alimentée par un flux (1) de norbornène brut, lequel titre environ 85 à 98% selon la qualité du DCPD ou du CPD/DCPD mis en réaction et les conditions de fonctionnement de la synthèse (rapport molaire, débit). Les impuretés, dont on trouvera une liste dans les Tableaux 1 et 2 ci-après, sont classées en :
- impuretés légères représentant 0,1 - 1% ;
- impuretés mi-lourdes représentant 0,1 - 1% ; et
- impuretés lourdes représentant 1 - 15% (pour 100% au total).

Cette colonne (C1) travaille à la pression atmosphérique, avec une température de pied de 100 à 160°C et une température de tête, de 95 à 110°C.

En tête de colonne (C1), on obtient un flux (2) ayant la composition suivante (pour 100%) :
- 0,1 - 2% de légers ;
- 0,1 - 2% de mi-lourds ; et
- 0 - 0,2% de lourds,
le restant étant constitué par du norbornène.

En pied de colonne (C1), on élimine un flux (3) ayant la composition suivante (pour 100%):
- 0 - 1% de légers ;
- 1 - 5% de mi-lourds ; et
- 50 - 80% de lourds,
le restant étant constitué par du norbornène.

Cette colonne (C1) permet d'éliminer, par exemple, dans le flux (3), environ 25 - 75% des mi-lourds méthylnorbornènes, qui sont dus au méthylcyclopentadiène, impureté classique et inévitable du CPD, et environ 95-99% des lourds (recombinaison du CPD en DCPD).

### Colonne (C2) et Réservoir (R)

La colonne (C2), garnie d'éléments de remplissage, a pour but d'éliminer la majorité des légers.

Cette colonne travaille à la pression atmosphérique, avec une température de pied de 95 - 100°C et une température de tête de 94 - 100°C.

En tête, on obtient un flux (4) ayant la composition suivante (pour 100%):
- 2 - 10% de légers ;
- 0 0,2% de mi-lourds ; et
- 0 - 0,5% de lourds,
le restant étant constitué par du norbornène.

Le flux (6) sortant du réservoir (R) en aval de la colonne (C2), a la composition suivante (pour 100%) :
- 0 - 0,1% de légers ;
- 0 - 0,5% de mi-lourds ; et
- 0 - 0,1% de lourds,
le restant étant constitué par du norbornène.

### Colonne (C3)

Cette colonne, garnie d'éléments de remplissage, a pour but d'éliminer le reste des lourds et des mi-lourds (méthylnorbornènes).

Elle travaille à la pression atmosphérique, avec une température de pied de 95 - 100°C et avec une température de tête de 95 - 100°C.

En tête, on obtient le flux (7) recherché, ayant la composition suivante (pour 100%) :
- 0 - 0,1% de légers ;
- 0 - 0,5% de mi-lourds ; et
- 0 - 0,1% de lourds,
Le restant étant constitué par du norbornène.

En pied, on obtient le flux (8) des lourds et mi-lourds, que l'on recycle dans le flux (1) et qui a la composition suivante (pour 100%):
- 0 - 0,1% de légers ;
- 1 - 10% de mi-lourds ; et
- 0 - 0,5% de lourds,
le restant étant constitué par du norbornène.

Ainsi, le procédé selon la présente invention permet d'obtenir une qualité de norbornène meilleure que les procédés antérieurs, avec l'avantage complémentaire que cette pureté supérieure est obtenue avec peu de pertes.

Les Exemples suivants illustrent la présente invention. Dans ces Exemples, les pourcentages sont également donnés en poids.

### Exemple 1

Dans la colonne (C1), fonctionnant à la pression atmosphérique, à une température de pied de 132°C et de tête de 98°C, on envoie 600 kg/h d'un flux (1) dont la composition est donnée dans le Tableau 1. En tête de colonne (C1), on renvoie une partie du flux distillé de façon à assurer un taux de soutirage de 0,86.

Les compositions des flux de tête (2) (564 kg/h) et de pied (3) (36 kg/h) sont également données dans le Tableau 1.

Le flux (2) (564 kg/h) est adressé à la colonne de distillation (C2), fonctionnant à la pression atmosphérique, à une température de tête de 94°C et à une température de pied de 97°C. En tête de colonne (C2), on renvoie une partie du flux distillé, de façon à assurer un taux de reflux/soutirage de 20,97.

La composition du flux de tête (4) (31 kg/h) est également donnée dans le Tableau 1.

Le flux (6) sortant du réservoir (R) (533 kg/h) a une composition également donnée dans le Tableau 1.

Le flux (6) est adressé à la colonne de distillation (C3) , fonctionnant à la pression atmosphérique, à une température de tête de 98°C et à une température de pied de 99°C. En tête de colonne (C3), on renvoie une partie du flux distillé de façon à assurer un taux de reflux/soutirage de 1,67.

Les compositions du flux de tète (7) (430 kg/h) et du flux de pied (8) (103 kg/h) sont données dans le Tableau 1.

### Exemple 2

Dans la colonne (C1), fonctionnant à la pression atmosphérique, à une température de pied de 130°C et de tête de 99°C, on envoie 740 kg/h d'un flux (1) dont la composition est donnée dans le Tableau 2.

En tête de colonne (C1), on renvoie une partie du flux distillé de façon à assurer un taux de soutirage de 0,89.

Les compositions des flux de tête (2) (682 kg/h) et de pied (3) (58 kg/h) sont également données dans le Tableau 2.

Le flux (2) (682 kg/h) est adressé à la colonne de distillation (C2), fonctionnant à la pression atmosphérique, à une température de tête de 95,5°C et à une température de pied de 95,5°C. En tête de colonne (C2), on renvoie une partie du flux distillé, de façon à assurer un taux de reflux/soutirage de 26,79.

La composition du flux de tête (4) (28 kg/h) est également donnée dans le Tableau 2.

Le flux (6) sortant du réservoir (R) (654 kg/h) a une composition également donnée dans le Tableau 2.

Le flux (6) est adressé à la colonne de distillation (C3), fonctionnant à la pression atmosphérique, à une température de tête de 99°C et à une température de pied de 100°C. En tête de colonne (C3), on renvoie une partie du flux distillé de façon à assurer un taux de reflux/soutirage de 1,13.

Les compositions du flux de tête (7) (610 kg/h) et du flux de pied (8) (44 kg/h) sont données dans le Tableau 2.

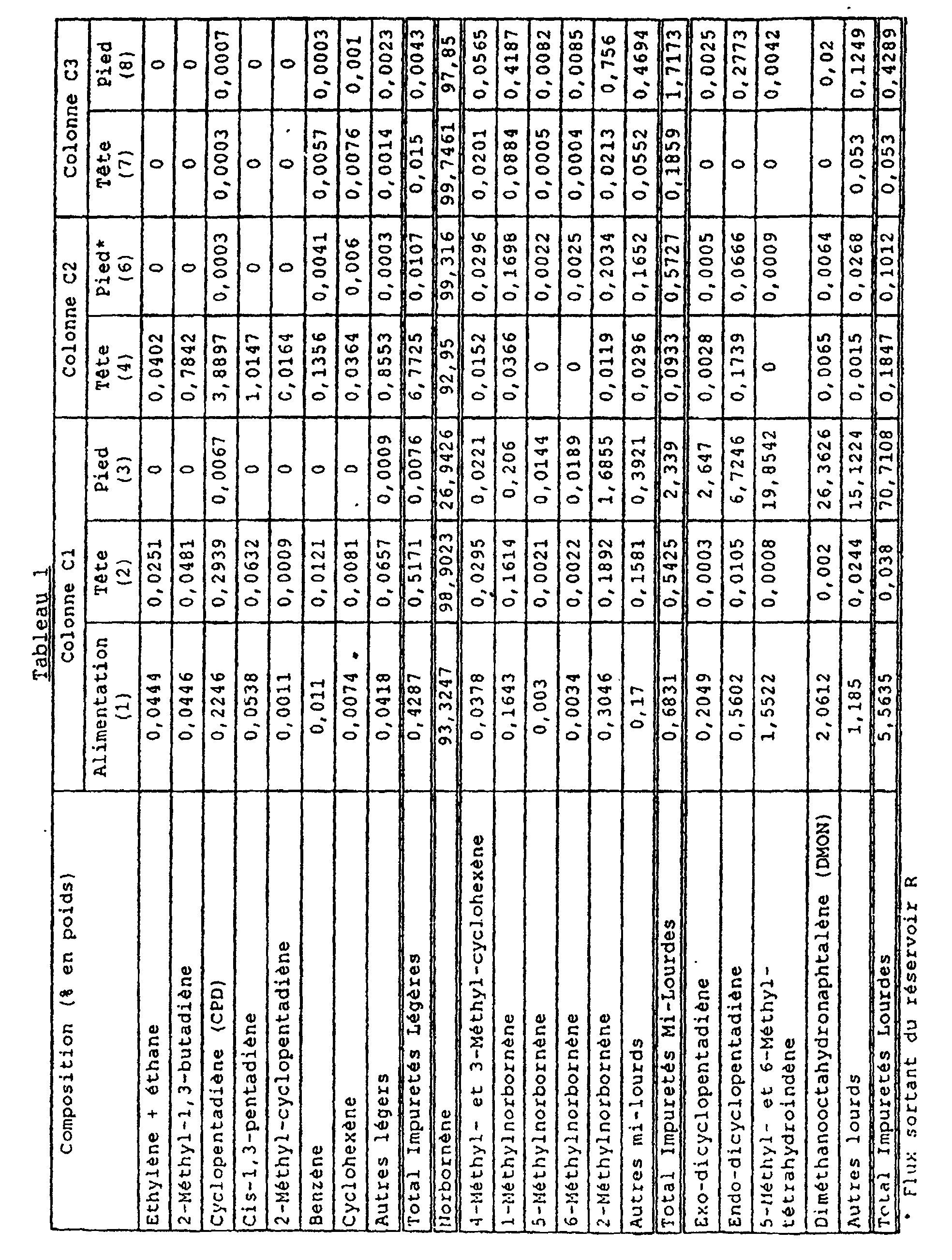

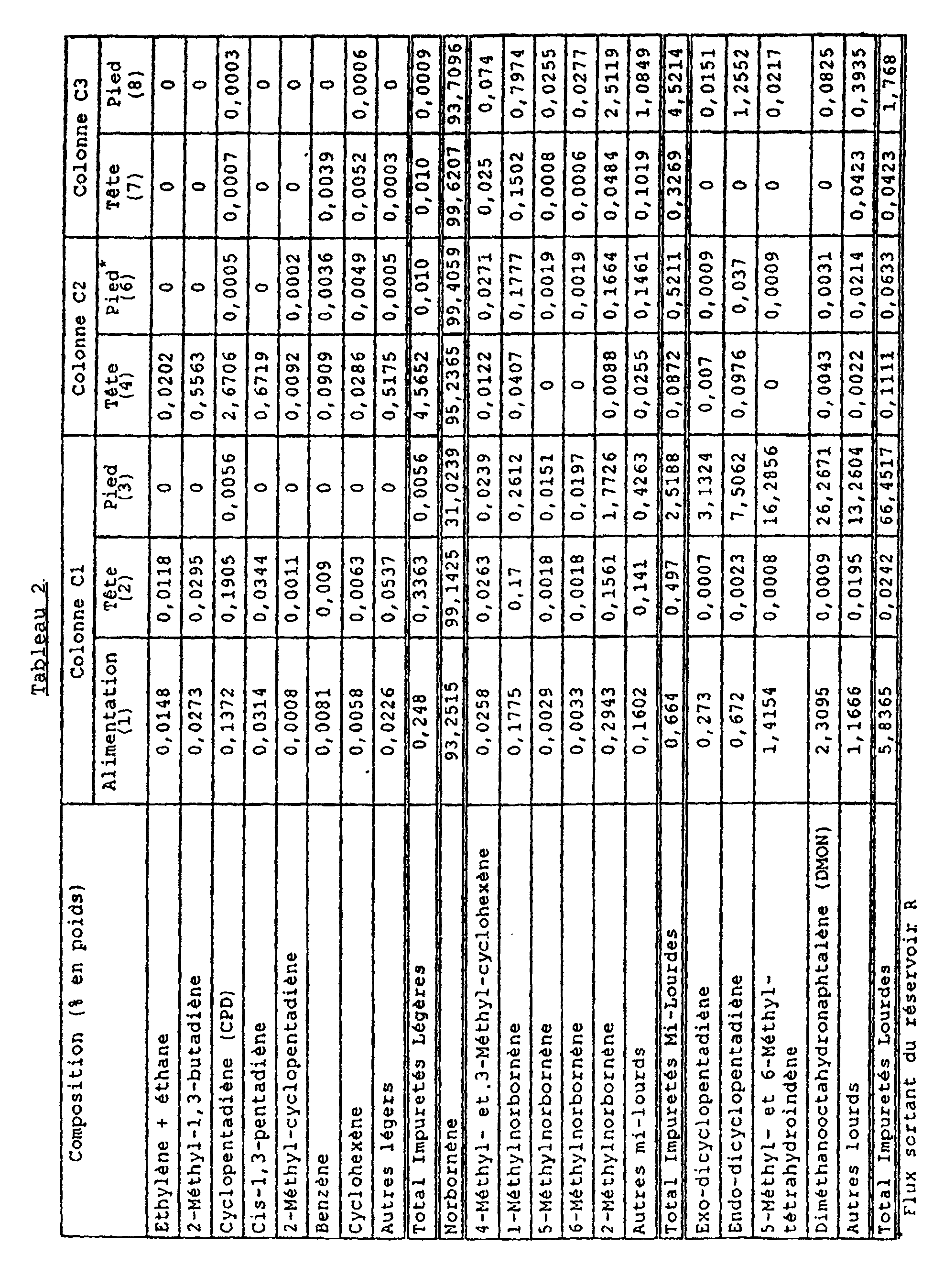

## Revendications

1. Procédé de purification du norbornène obtenu par la réaction du dicyclopentadiène ou du cyclopentadiène et de l'éthylène, le mélange brut réactionnel contenant :
- des impuretés légères, dont les températures d'ébullition sont inférieures à celle du norbornène ;
- des impuretés mi-lourdes, dont les températures sont comprises entre celle du norbornène et celle de l'éthyl norbornène; et
- des impuretés lourdes, dont les températures sont supérieures à celles de l'éthyl norbornène, celui-ci étant par ailleurs inclus dans lesdites impuretés lourdes,
**caractérisé par le fait qu'**on effectue une première distillation du mélange brut réactionnel dans une colonne d'équeutage (C1), éliminant une partie des impuretés lourdes et une partie des impuretés mi-lourdes ; puis une seconde distillation du mélange brut ainsi équeuté dans une colonne d'étêtage (C2), éliminant les impuretés légères ; et ensuite une troisième distillation du mélange ainsi étêté dans une colonne d'équeutage (C3), éliminant le reste des impuretés lourdes et mi-lourdes.

2. Procédé selon la revendication 1, **caractérisé par le fait qu'**on poursuit, dans un réservoir (R) interposé entre le colonne d'étêtage (C2) et la colonne de second équeutage (C3), la recombinaison du cyclopentadiène en dicyclopentadiène qui commence dans la colonne d'équeutage (C1) et se poursuit dans la colonne d'étêtage (C2).

3. Procédé selon l'une des revendications 1 et 2, **caractérisé par le fait que** l'on fait fonctionner la colonne de premier équeutage (C1) à la pression atmosphérique, à une température de pied de 100 à 160°C et une température de tête de 95 à 110°C.

4. Procédé selon l'une des revendications 1 à 3, **caractérisé par le fait que** l'on fait fonctionner la colonne d'étêtage (C2) à la pression atmosphérique, à une température de pied de 95 à 100°C et à une température de tête de 94 à 100°C.

5. Procédé selon l'une des revendications 1 à 4, **caractérisé par le fait que** l'on fait fonctionner la colonne de second équeutage (C3) à la pression atmosphérique, à une température de pied de 95 à 100°C et à une température de tête de 95 à 100°C.

6. Procède selon l'une des revendications 1 à 5, **caractérisé par le fait que** l'on recycle à l'entrée de la colonne de premier équeutage (C1) le flux de pied de la colonne de second équeutage (C3).

7. Procédé selon l'une des revendications 1 à 6, **caractérisé par le fait qu'**on alimente la colonne d'équeutage (C1) par un flux (1) de norbomène brut ayant de composition :
- 0,1-1% d'impuretés légères ;
- 0,1-1% d'impuretés mi-lourdes ;
- 1-15% d'impuretés lourdes,
le restant étant constitué par du norbornène,
pour séparer :
- en tête, un flux (2) de composition :
. 0,1 à 2% d'impuretés légères ;
. 0,1 à 2% d'impuretés mi-lourdes ;
. 0 à 0.2% d'impuretés lourdes,
le restant étant constitué par du norbornène ; et
- en pied, un flux (3) de composition :
. 0 à 1% d'impuretés légères ;
. 1 à 5% d'impuretés mi-lourdes ;
. 50 à 80% d'impuretés lourdes,
le restant étant constitué par du norbornène ;
puis qu'on envoie le flux (2) dans la colonne d'étêtage (C2) pour séparer :
- en tète, un flux (4) de composition :
. 2 à 10% d'impuretés légères ;
. 0 à 0,2% d'impuretés mi-lourdes;
. 0 à 0,5% d'impuretés lourdes,
le restant étant constitué par du norbornène ; et
- en pied, un flux (6) de composition :
. 0 à 0,1 % d'impuretés légères ;
. 0 à 0,5% d'impuretés mi-lourdes ;
. 0 à 0,1% d'impuretés lourdes,
le restant étant constitué par du norbornène ;
puis qu'on envoie le flux (6) dans la seconde colonne d'équeutage (C3) pour séparer :
- en tête, le flux (7) recherché de composition :
. 0 à 0,1% d'impuretés légères ;
. 0 à 0,5% d'impuretés mi-lourdes ;
. 0 à 0,1 % d'impuretés lourdes,
le restant étant constitué par du norbornène ; et
- en pied, un flux (8) de composition :
. 0 à 0,1% d'impuretés légères ;
. 1 à 10% d'impuretés mi-lourdes;
. 0 à 0,5% d'impuretés lourdes,
le restant étant constitué par du norbornène,
les pourcentages étant en poids et toutes les compositions étant indiquées pour un total de 100%.

## Claims

1. Process for the purification of the norbornene obtained by the reaction of dicyclopentadiene or cyclopentadiene and ethylene, the crude reaction mixture comprising:
- light impurities, the boiling temperatures of which are lower than that of norbornene;
- medium-heavy impurities, the boiling temperatures of which are between that of norbornene and that of ethylnorbornene; and
- heavy impurities, the boiling temperatures of which are greater than that of ethylnorbornene, the latter furthermore being included in the said heavy impurities,
**characterized in that** a first distillation of the crude reaction mixture is carried out in a tailing column (C1), removing a portion of the heavy impurities and a portion of the medium-heavy impurities; then a second distillation of the crude mixture, thus tailed, is carried out in a topping column (C2), removing the light impurities; and a third distillation of the mixture, thus topped, is subsequently carried out in a tailing column (C3), removing the remainder of the heavy and medium-heavy impurities.

2. Process according to Claim 1, **characterized in that** the recombination of the cyclopentadiene to dicyclopentadiene, which begins in the tailing column (C1) and is continued in the topping column (C2), is continued in a tank (R) interposed between the topping column (C2) and the second tailing column (C3).

3. Process according to either of Claims 1 and 2, **characterized in that** the first tailing column (C1) is operated at atmospheric pressure, at a bottom temperature of 100 to 160°C and at a top temperature of 95 to 110°C.

4. Process according to one of Claims 1 to 3, **characterized in that** the topping column (C2) is operated at atmospheric pressure, at a bottom temperature of 95 to 100°C and at a top temperature of 94 95 to 100°C and at a top temperature of 94 to 100°C.

5. Process according to one of Claims 1 to 4, **characterized in that** the second tailing column (C3) is operated at atmospheric pressure, at a bottom temperature of 95 to 100°C and at a top temperature of 95 to 100°C.

6. Process according to one of Claims 1 to 5, **characterized in that** the bottom flow from the second tailing column (C3) is recycled at the inlet of the first tailing column (C1).

7. Process according to one of Claims 1 to 6, **characterized in that** the tailing column (C1) is fed with a flow (1) of crude norbornene with the composition:
• 0.1 - to of light impurities;
• 0.1 - 1% of medium-heavy impurities;
• 1-15% of heavy impurities,
the remainder being composed of norbornene,
in order to separate:
- at the top, a flow (2) with the composition:
• 0.1 to 2% of light impurities;
• 0.1 to 2% of medium-heavy impurities;
• 0 to 0.2% of heavy impurities,
the remainder being composed of norbornene; and
- at the bottom, a flow (3) with the composition:
• 0 to 1% of light impurities;
• 1 to 5% of medium-heavy impurities;
• 50 to 80% of heavy impurities,
the remainder being composed of norbornene;
**in that** the flow (2) is then conveyed into the topping column (C2),
in order to separate:
- at the top, a flow (4) with the composition:
• 2 to 10% of light impurities;
• 0 to 0.2% of medium-heavy impurities;
• 0 to 0.5% of heavy impurities,
the remainder being composed of norbornene; and
- at the bottom, a flow (6) with the composition:
• 0 to 0.1% of light impurities;
• 0 to 0.5% of medium-heavy impurities;
• 0 to 0.1% of heavy impurities,
the remainder being composed of norbornene;
and **in that** the flow (6) is then conveyed into the second tailing column (C3),
in order to separate:
- at the top, the desired flow (7) with the composition:
• 0 to 0.1% of light impurities;
• 0 to 0.5% of medium-heavy impurities;
• 0 to 0.1% of heavy impurities,
the remainder being composed of norbornene; and
- at the bottom, a flow (8) with the composition:
• 0 to 0.1% of light impurities;
• 1 to 10% of medium-heavy impurities;
• 0 to 0.5% of heavy impurities,
the remainder being composed of norbornene,
the percentages being by weight and all the compositions being indicated for a total of 100%.

## Patentansprüche

1. Verfahren zur Reinigung von Norbornen, das durch die Umsetzung von Dicyclopentadien oder Cyclopentadien mit Ethylen erhalten wird, wobei das erhaltene Rohgemisch enthält:
- niedrigsiedende Verunreinigungen, deren Siedetemperaturen unterhalb der Siedetemperatur von Norbornen liegen,
- mittelsiedende Verunreinigungen, deren Siedetemperaturen im Bereich von der Siedetemperatur von Norbornen bis zur Siedetemperatur von Ethylnorbornen liegen, und
- hochsiedende Verunreinigungen, deren Siedetemperaturen oberhalb der Siedetemperatur von Ethylnorbornen liegen, das selbst im übrigen in diesen hochsiedenden Verunreinigungen enthalten ist,
**dadurch gekennzeichnet, daß** eine erste Destillation des Rohgemischs in einer Kolonne (C1) zur Abtrennung von Sumpfprodukt durchgeführt wird, bei der ein Teil der hochsiedenden Verunreinigungen und ein Teil der mittelsiedenden Verunreinigungen entfernt werden, wonach eine zweite Destillation dieses Rohgemischs, aus dem Sumpfprodukt abgetrennt wurde, in einer Kolonne (C2) zur Abtrennung von Kopfprodukt durchgeführt wird, bei der die niedrigsiedenden Verunreinigungen entfernt werden, wonach eine dritte Destillation dieses Gemischs, aus dem Kopfprodukt abgetrennt wurde, in einer Kolonne (C3) zur Abtrennung von Sumpfprodukt durchgeführt wird, bei der die restlichen hochsiedenden Verunreinigungen und mittelsiedenden Verunreinigungen entfernt werden.

2. Verfahren nach Anspruch 1, **dadurch gekennzeichnet, daß** in einem Reservoir (R), das zwischen der Kolonne (C2) zur Abtrennung von Kopfprodukt und der Kolonne (C3) zur zweiten Abtrennung von Sumpfprodukt angeordnet ist, die Rekombination des Cyclopentadiens zu Dicyclopentadien fortgesetzt wird, die in der Kolonne (C1) zur Abtrennung von Sumpfprodukt beginnt und in der Kolonne (C2) zur Abtrennung von Kopfprodukt fortgesetzt wird.

3. Verfahren nach einem der Ansprüche 1 und 2, **dadurch gekennzeichnet, daß** die Kolonne (C1) für die erste Abtrennung von Sumpfprodukt bei Atmosphärendruck, einer Sumpftemperatur von 100 bis 160 °C und einer Kopftemperatur von 95 bis 110 °C betrieben wird.

4. Verfahren nach einem der Ansprüche 1 bis 3, **dadurch gekennzeichnet, daß** die Kolonne (C2) zur Abtrennung von Kopfprodukt bei Atmosphärendruck, einer Sumpftemperatur von 95 bis 100 °C und einer Kopftemperatur von 94 bis 100 °C betrieben wird.

5. Verfahren nach einem der Ansprüche 1 bis 4, **dadurch gekennzeichnet, daß** die Kolonne (C3) für die zweite Abtrennung von Sumpfprodukt bei Atmosphärendruck, einer Sumpftemperatur von 95 bis 100 °C und einer Kopftemperatur von 95 bis 100 °C betrieben wird.

6. Verfahren nach einem der Ansprüche 1 bis 5, **dadurch gekennzeichnet, daß** der Strom vom Sumpf der Kolonne (C3) für die zweite Abtrennung von Sumpfprodukt zum Einlaß der Kolonne (C1) für die erste Abtrennung von Sumpfprodukt zurückgeleitet wird.

7. Verfahren nach einem der Ansprüche 1 bis 6, **dadurch gekennzeichnet, daß** die Kolonne (C1) zur Abtrennung von Sumpfprodukt mit dem Strom (1) aus dem als Rohprodukt erhaltenen Norbornen versorgt wird, der die folgende Zusammensetzung hat:
• 0,1 bis 1 % niedrigsiedende Verunreinigungen,
• 0,1 bis 1 % mittelsiedende Verunreinigungen,
• 1 bis 15 % hochsiedende Verunreinigungen,
wobei der Rest aus Norbornen besteht,
um abzutrennen:
- am Kopf einen Strom (2) mit der folgenden Zusammensetzung:
• 0,1 bis 2 % niedrigsiedende Verunreinigungen,
• 0,1 bis 2 % mittelsiedende Verunreinigungen,
• 0 bis 0,2 % hochsiedende Verunreinigungen,
wobei der Rest aus Norbornen besteht; und
- vom Sumpf einen Strom (3) mit der folgenden Zusammensetzung:
• 0 bis 1 % niedrigsiedende Verunreinigungen,
• 1 bis 5 % mittelsiedende Verunreinigungen,
• 50 bis 80 % hochsiedende Verunreinigungen,
wobei der Rest aus Norbornen besteht;
wonach der Strom (2) in die Kolonne (C2) zur Abtrennung von Kopfprodukt geleitet wird, um abzutrennen:
- am Kopf einen Strom (4) mit der folgenden Zusammensetzung:
• 2 bis 10 % niedrigsiedende Verunreinigungen,
• 0 bis 0,2 % mittelsiedende Verunreinigungen,
• 0 bis 0,5 % hochsiedende Verunreinigungen,
wobei der Rest aus Norbornen besteht; und
- vom Sumpf einen Strom (6) mit der folgenden Zusammensetzung:
• 0 bis 0,1 % niedrigsiedende Verunreinigungen,
• 0 bis 0,5 % mittelsiedende Verunreinigungen,
• 0 bis 0,1 % hochsiedende Verunreinigungen,
wobei der Rest aus Norbornen besteht;
wonach der Strom (6) in die zweite Kolonne (C3) zur Abtrennung von Sumpfprodukt geleitet wird, um abzutrennen:
- am Kopf den Produktstrom (7) mit der folgenden Zusammensetzung:
• 0 bis 0,1 % niedrigsiedende Verunreinigungen,
• 0 bis 0,5 % mittelsiedende Verunreinigungen,
• 0 bis 0,1 % hochsiedende Verunreinigungen,
wobei der Rest aus Norbornen besteht; und
- vom Sumpf einen Strom (8) mit der folgenden Zusammensetzung:
• 0 bis 0,1 % niedrigsiedende Verunreinigungen,
• 1 bis 10 % mittelsiedende Verunreinigungen,
• 0 bis 0,5 % hochsiedende Verunreinigungen,
wobei der Rest aus Norbornen besteht;
wobei die Prozentangaben Angaben in Gewichtsprozent sind und alle Zusammensetzungen für eine Gesamtmenge von 100 % angegeben sind.
